Europäisches Patentamt

European Patent Office

Office européen des brevets

(19)

(11) Veröffentlichungsnummer: **0 236 985 B1**

(12) # EUROPÄISCHE PATENTSCHRIFT

(45) Veröffentlichungstag der Patentschrift: **22.07.92**

(21) Anmeldenummer: **87103255.3**

(22) Anmeldetag: **06.03.87**

(51) Int. Cl.5: **C12Q 1/56**, G01N 33/86, G01N 33/68, C07K 5/08, C07K 5/10

(54) **Verfahren zur photometrischen Bestimmung der Protein C- und/oder Protein S-Aktivität.**

(30) Priorität: **07.03.86 DE 3607559**

(43) Veröffentlichungstag der Anmeldung:
**16.09.87 Patentblatt 87/38**

(45) Bekanntmachung des Hinweises auf die Patenterteilung:
**22.07.92 Patentblatt 92/30**

(84) Benannte Vertragsstaaten:
**AT BE CH DE ES FR GB GR IT LI LU NL SE**

(56) Entgegenhaltungen:
**EP-A- 0 003 474      EP-A- 0 014 039**
**EP-A- 0 076 042      EP-A- 0 137 269**
**EP-A- 0 182 929      EP-A- 0 203 509**
**EP-A- 0 229 234**

**CHEMICAL ABSTRACTS, Band 100, Nr. 19, 07 Mai 1984, Columbus, OH (US); N.SALA et al., Seite 216, Nr. 152824t**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Bartl, Knut, Dr.**
**Am Westend 6**
**W-8121 Wilzhofen(DE)**
Erfinder: **Dessauer, Andreas, Dr.**
**Herre-Str. 1**
**W-8132 Tutzing(DE)**
Erfinder: **Lill, Helmut, Dr.**
**Zugspitzstr. 24**
**W-8121 Wielenbach(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach 86 08 20**
**W-8000 München 86(DE)**

**Beschreibung**

Die Erfindung betrifft ein Verfahren zur photometrischen Bestimmung von Protein C und/oder Protein S-Aktivität, im besonderen im Plasma.

Protein C ist ein zweikettiges, Vitamin K abhängiges Glykoprotein im Plasma. Seine Synthese erfolgt in der Leber. Dabei wird zunächst eine gerinnungsphysiologisch indifferente Vorstufe (Decarboxy-Protein C) gebildet. Carboxylierung von $\gamma$-Glutaminsäureresten im Protein durch eine Vitamin K-abhängige Carboxylase führt zum Protein C. Protein C selbst ist ein Proenzym und wird durch Thrombin in aktiviertes Protein C überführt. Letzteres wirkt durch die proteolytische Inaktivierung der aktivierten Gerinnungsfaktoren V und VIII als ein Antikoagulans. Die antikoagulatorische Wirkung des aktiven Protein C wird durch einen Cofaktor, Protein S, verstärkt. Protein S ist ein einkettiges, ebenfalls Vitamin K abhängiges Glykoprotein im Plasma. Aktives Protein C und Protein S bilden einen äquimolaren Komplex. Erniedrigte Protein-C-, wie auch Protein S Spiegel wurden bei Patienten mit Lebererkrankungen, Verbrauchs-Coagulopathie (DIC) und nach Warfarintherapie beschrieben. Ein angeborener Mangel an Protein C oder an Protein S führt zu venösen thromboembolischen Risiken. Protein C, wie auch Protein S spielten daher eine wichtige Rolle sowohl bei der physiologischen Hämostasis, als auch in vielen Krankheiten, im besonderen bei der Thrombosis.

Bei einem bislang im Handel erhältlichen Testverfahren wird die Menge an Protein C im Plasma durch enzymmarkierte Antikörper bestimmt. Dieses Verfahren hat jedoch den Nachteil, daß die zur Bestimmung eingesetzten Antikörper auch mit dem oben erwähnten Decarboxyprotein C reagieren. Da die Plasmakonzentration an Decarboxyprotein während einer Behandlung mit Antikoagulantien häufig stark ansteigt, birgt dieses Verfahren eine große Fehlerquelle, die unter Umständen zu einer falschen oder unzureichenden Therapie führen kann.

Der Nachteil bei der Verwendung immunologischer Bestimmungsverfahren im Plasma für Protein C liegt darin, daß sie keine Information über die biologische Aktivität der Protein-C-Moleküle liefern. Die Präsenz von abnormalem Protein C mit stark reduzierter biologischer Aktivität (genetische Variante) kann mit solchen Verfahren nicht gefunden werden.

R. B. Francis und M. J. Patch (Thrombosis Research 32, 605-613, 1983) lehren ein Verfahren zur Bestimmung von aktiviertem Protein C aus Humanplasma durch Bestimmung der Partialthromboplastinzeit (PTT-Test). Dabei wird das durch Adsorption an Bariumcitrat von Plasma abgetrennte Protein C durch Zusatz von Thrombin aktiviert und danach letzteres durch einen Überschuß an Antithrombin III und Heparin inhibiert. Heparin wiederum wird durch eine jeweils neu zu bestimmende exakte Menge von Protaminsulfat neutralisiert. Danach wird Protein C über die partielle Thromboplastinzeit bestimmt werden. Indikatorreaktion ist hier die durch Thrombin bewirkte Spaltung von Fibrinogen und Bildung eines Fibrin-Gerinnsels.

R. M. Bertina et al (Thromb. Haemostas 51, (1) 1-5, (1984)) lehren ein spektrophotometrisches Verfahren zur Bestimmung von Protein C-Aktivität. Dieses Verfahren umfaßt drei unabhängige Schritte:

1. Isolierung des Protein C mit Hilfe einer Al(OH)$_3$-Adsorption.
2. Aktivierung des vom Plasma abgetrennten Protein C mit Thrombin, sowie nachfolgende Inhibierung des letzteren durch äquimolare Mengen Antithrombin III und Heparin.
3. Messen der proteolytischen Aktivität von isoliertem aktiviertem Protein C mit einem chromogenen Substrat (S$_{2366}$ = H pyro-Glu-Pro-Arg-pNA).

Dieses Verfahren ist unbefriedigend bezüglich der Spezifität für Protein C, da dieses Substrat auch von anderen Gerinnungsproteasen gespalten wird. Es kann somit zu falsch positiven Ergebnissen kommen.

In einer weiteren photometrischen Bestimmungsmethode wird der Protein C-Aktivator Protac® - (Hersteller Pentapharm, Schweiz), welcher aus dem Gift der Schlange Agkistrodon contortrix contortrix gewonnen wird, sowie ein chromogenes Protein C-Substrat (2 AcOH.H-Pro-Pro-Arg-pNA) verwendet. Bei diesem Verfahren kann auf die Probenvorbereitung verzichtet werden. Mit diesem Verfahren kann jedoch nicht zwischen carboxyliertem und nicht carboxyliertem Protein C unterschieden werden. Es ist aber bekannt, daß nur carboxyliertes Protein C in vivo wirksam ist. Daher kann mit diesem Verfahren keine Information über die biologische Aktivität der Protein C-Moleküle erhalten werden.

Bei einem weiteren bekannten Verfahren zur Bestimmung von Protein C wird ebenfalls als Protein C-Aktivator Protac® verwendet. Die Protein C-Bestimmung erfolgt dabei nach Zusatz von Aktivatoren für das endogene Gerinnungssystem über einen Clotting-Test. Mit diesem Verfahren ist es zwar möglich, zwischen carboxyliertem und nicht carboxyliertem Protein C zu unterscheiden, jedoch hat dieses Verfahren den Nachteil, daß die Detektion über die Gerinnselbildung (Clotting) erfolgen muß. Damit ist die Bestimmung an bestimmte Detektionsverfahren (z. B. Häkelmethode oder Magnetfeldmethode) gebunden, die sich überdies als störanfällig und nicht automatisierbar erwiesen.

Gemäß einem älteren Vorschlag der gleichen Anmelderin erfolgt die PC-Bestimmung, indem zuerst unter Einwirkung von Thrombin aktiviertes Protein C gebildet wird, danach dieses Thrombin inaktiviert wird und anschließend durch die Gerinnungskaskade neu gebildetes Thrombin mit Hilfe eines synthetischen Thrombinsubstrates bestimmt wird. Tatsächlich gemessen wird dabei die Verlangsamung der Thrombinbildung im Vergleich zu einer Testdurchführung ohne Protein C.

Die Menge an Protein S im Plasma kann in einem immunoradiometrischen Test, wie z. B. von Bertina et al. (Thromb. Haemostas, 53 (2), 268-272 (1985)) beschrieben, bestimmt werden. Dieses Verfahren hat jedoch den Nachteil, daß die zur Bestimmung eingesetzten Antikörper auch mit nicht funktionellem Protein S, d. h. z. B. mit Decarboxy-Protein S oder mit von $C_{4b}$-bindendes Protein komplexiertem Protein S, reagieren. Dies führt unter Umständen zu falsch positiven Protein-S-Werten und damit zu unzureichender Therapie.

Im gleichen Artikel beschreiben die Autoren Bertina et al. auch eine Möglichkeit, wie funktionelles Protein S aus Humanplasma bestimmt werden kann: Wird zu einem Humanplasma aktiviertes Protein C gemischt und anschliessend die Partialthromboplastinzeit (PTT) dieser Mischung bestimmt, so ist die PTT umso länger, je größer die Konzentration an funktionellem Protein S ist. Indikatorreaktion ist auch hier die durch Thrombin bewirkte Spaltung von Fibrinogen und Bildung eines Fibrin-Gerinnsels. Das Verfahren ist somit an bestimmte Detektionverfahren gebunden, die störanfällig und im allgemeinen nicht automatisierbar sind. Es hat die weiteren Nachteile, daß gereinigtes, aktiviertes Protein C zugesetzt werden muß und daß Protein C und Protein S im Plasma nicht gleichzeitig bestimmt werden können.

In der älteren, nicht vorveröffentlichten EP-A-0 203 509 wird ein Verfahren zur quantitativen Bestimmung von Protein C beschrieben, bei welchem eine das Protein C enthaltende Probe mit dem Gift der Schlange Agkistrodon contortrix oder einem damit kreuzreagierenden Gift oder einem aus diesen Giften erhältlichen Aktivatorpräparat aktiviert und das gebildete aktivierte Protein C durch photometrische Messung der Menge oder der Bildungsgeschwindigkeit eines farbigen oder fluoreszierenden Spaltproduktes, welches das aktivierte Protein C aus einem synthetischen chromogenen Substrat bildet, oder der durch dieses bewirkte Verlängerung der Gerinnungszeit bestimmt.

Der Erfindung liegt die Aufgabe zugrunde, ein Verfahren zur photometrischen Bestimmung von biologisch wirksamem Protein C und/oder Protein S zu schaffen, welches einfacher durchführbar als die erwähnten Methoden und automatisierbar ist.

Gelöst wird diese Aufgabe, indem die das zu bestimmende Protein C oder Protein S enthaltende Probe mit einem Protein C-Aktivator aus Schlangengift unter Bildung von aktiviertem Protein C oder Protein S inkubiert wird, ein Aktivator für Faktor XII, für Faktor VII oder für Faktor II zugesetzt wird und die Abnahme der durch Gerinnungsfaktoren und deren Aktivatoren vermittelten Bildung von Thrombin aus Prothrombin mittels eines chromogenen Thrombinsubstrates bestimmt wird.

Die Erfindung baut auf dem überraschenden Befund auf, daß Protein C-Aktivator aus Schlangengift im Gegensatz zu anderen Protein-C-Aktivatoren Thrombinsubstrate nicht spaltet.

Dies war deshalb überraschend, weil z. B. Protac® ein in seiner Wirkung thrombinähnlicher Aktivator für Protein C ist. Demzufolge war zu erwarten, daß das chromogene Thrombinsubstrat auch vom Schlangengift-Aktivator bzw. der Mischung des Aktivators mit Protein C umgesetzt wird, denn Thrombin und die sonst bekannten Protein C-Aktivatoren (z. B. Plasmin, Trypsin) setzen chromogene Thrombinsubstrate um. Wenn derartige Protein C-Aktivatoren eingesetzt werden, müßte deshalb nach Aktivierung von Protein C der Aktivator inaktiviert werden. Erst anschließend könnten die Aktivatoren für Faktor XII, VII oder II und das chromogene Thrombinsubstrat zugesetzt werden. Bei Verwendung eines Protein C-Aktivators aus Schlangengift zeigt sich jedoch, daß überraschenderweise auf diesen Inaktivierungsschritt verzichtet werden kann. Es ist sogar möglich, die genannten Aktivatoren und das chromogene Thrombinsubstrat bereits bei Beginn der Bestimmung zuzusetzen.

Als Protein C-Aktivator können im Rahmen der Erfindung Lösungen von Schlangengift, z. B. von Agkistrodon contortrix contortrix, A.C. mokasen, A.C. pictigaster, A.piscivours, A.p. leucostoma, A. bilineatus, Bothrops moojeni, B. pradoi, Cerastes cerastses, Vipera lebetrina oder V. russellii verwendet werden. Bevorzugt wird jedoch der aus dem Schlangengift der Agkistrodon contortrix contortrix isolierte Protein C-Aktivator verwendet.

Bevorzugt werden Gifte oder Giftkomponenten verwendet, die auch in Plasma nur Protein-C aktivieren, wobei die gewünschten Giftkomponenten leicht aus dem Gift einer Schlange getrennt werden können.

Hierzu können die allgemein üblichen Verfahren, wie Anionenaustauscher-Chromatographie, Affinitätschromatographie mit Protein C oder/ und Ultrafiltration angewendet werden.

Der Aktivator wird in einer Konzentration von 0,05 bis 5 U/ml, bevorzugt von 0,5 bis 1 U/ml Aktivator-Lösung eingesetzt. Hierbei ist eine Einheit (U) Protein C-Aktivator definiert als die Menge, welche die in 1 ml normalem menschlichem Citrat-Plasma enthaltene Menge Protein C bei, 37°C, pH 7 bis 8, in einer Reaktionsmischung aus einem Volumenteil Plasma und 4 bis 8 Volumenteilen wäßriger Protein C-Aktivatorlösung vollständig aktiviert.

3

Da aktiviertes Protein C (APC) die Gerinnungsfaktoren V und VIII proteolytisch inaktiviert, wobei Protein S als Cofaktor fungiert, wird beim erfindungsgemäßen Verfahren das Gerinnungssystem durch Zusatz der erwähnten Aktivatoren selbst derart modifiziert, daß sich die Inaktivierung der Faktoren V bzw. VIII in einer möglichst ausgeprägten Verringerung der Thrombinbildung auswirkt. Gemäß einer ersten Ausführungsform des erfindungsgemäßen Verfahrens wird dies zweckmäßig dadurch erreicht, daß man einen Aktivator für Faktor XII, beispielsweise Ellagsäure unter Zusatz von Kephalin, zusetzt. Gemäß einer zweiten Ausführungsform gibt man einen Aktivator für Faktor VII, z.B. Thromboplastin, und Faktor V zu. Gemäß einer dritten Ausführungsform setzt man als Aktivator für Faktor II Faktor Xa unter Zusatz von Kephalin zu. Üblicherweise enthalten die hierbei verwendeten Puffer noch zusätzlich Calciumionen.

Diese Ausführungsformen der Erfindung berücksichtigen die Tatsache, daß die Konzentrationen der Gerinnungsparameter Faktoren XII, XI, VIII, X, V und II die Zeit der Thrombinbildung beeinflussen derart, daß ein bestimmter Thrombinschwellenwert umso eher erreicht wird, je höher die Konzentration dieser Faktoren ist.

Die Thrombinbildung wird im Rahmen der Erfindung nach an sich bekannten Methoden bestimmt. Geeignet hierfür ist die partielle Thromboplastinzeit (PTT)-Methode. Bei ihrer Durchführung wird zweckmäßig ein Aktivator für Faktor XII zugesetzt. Die Thrombinbildung kann auch nach der Prothrombinzeit-Methode bestimmt werden. In diesem Falle setzt man zweckmäßig einen Aktivator für Faktor VII und F Va zu.

Als chromogenes Thrombinsubstrat kann im Rahmen der Erfindung jedes für die Thrombinbestimmung geeignete chromogene Substrat verwendet werden. Bevorzugt wird jedoch im Rahmen der Erfindung H-D-Phe-Pip-Arg-pNA oder Tos-Gly-Pro-Arg-pNA verwendet, wobei pNA = para-Nitroanilin bedeutet. Das pNA stellt den chromogenen Bestandteil der Substrate dar und wird durch das gebildete Thrombin abgespalten, so daß es photometrisch in bekannter Weise bestimmt werden kann.

Als Gerinnungssystem kann beispielsweise eine Mischung der Faktoren II bis XII oder Substratplasma (z. B. Protein C- bzw. Protein S-Mangelplasma) eingesetzt werden.

Im Rahmen der Erfindung kann jedes Plasma eingesetzt werden, bevorzugt wird Citratplasma.

Das Verfahren kann bei neutralen oder schwach alkalischen pH-Werten durchgeführt werden, vorzugsweise zwischen pH 6 und 9. Als Puffer können die in diesem Bereich wirksamen physiologisch unbedenklichen Puffer verwendet werden wie z. B. Tris/HCl. Ferner können die für Gerinnungstests üblichen Stabilisatoren und Konservierungsmittel wie Rinderserumalbumin, Merthiolat und dergleichen, ebenfalls zugesetzt werden.

Mit dem erfindungsgemäßen Verfahren können Protein C und Protein S gemeinsam oder Protein C bzw. Protein S jeweils allein bestimmt werden.

Wenn Protein C und Protein S gemeinsam bestimmt werden sollen, kann das Patientenplasma direkt zur Bestimmung eingesetzt werden. Wenn Protein C allein bestimmt werden soll, muß außer dem Reagenz noch Protein S zugesetzt werden. Vorteilhaft wird dies in der Form eines Protein C-Mangelplasmas erfolgen, welches Protein S enthält. Die Menge des zugesetzten Protein S muß dabei mindestens so groß sein wie die vermutete Protein-C-Konzentration. Wenn Protein S allein bestimmt werden soll, muß außer Reagenz noch Protein C zugesetzt werden. Vorteilhaft wird dies in Form eines Protein S-Mangelplasmas erfolgen. Auch hier muß mindestens die gleiche Menge Protein C zugesetzt werden, wie die vermutete Protein S-Konzentration beträgt.

Protein S bzw. Protein C-Mangelplasma sind herstellbar durch Immunadsorptionschromatographie, wie beispielsweise in Bertina, R.M., et al. Thromb. Haemostas (Stuttgart) 51, 1-5 (1984) bzw. in Bertina, R.M., et al. Thrombosis and Haemostasis 53, 268-272 (1985) beschrieben.

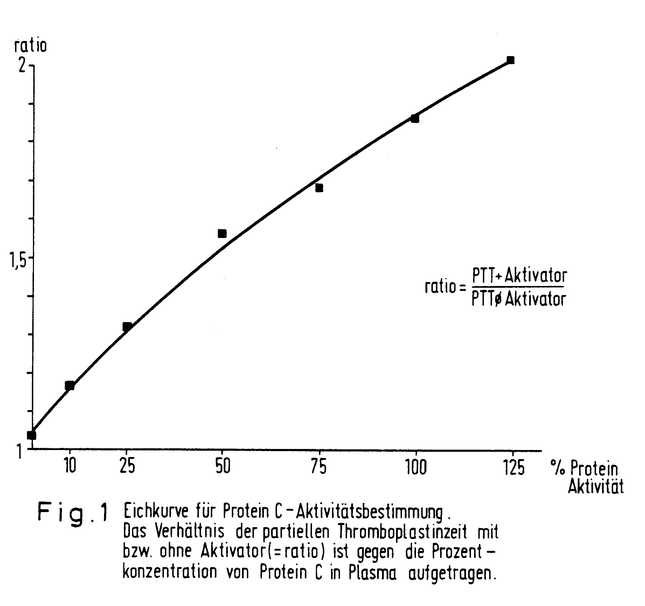

Die folgenden Beispiele erläutern die Erfindung weiter, in Verbindung mit der beigefügten Zeichnung (Fig. 1). In dieser ist dargestellt: Eine Eichkurve für Protein C-Aktivitätsbestimmung. Das Verhältnis der partiellen Thromboplastinzeit mit bzw. ohne Aktivator (Ratio) ist gegen Prozentkonzentration von Protein C im Plasma aufgetragen.

**Beispiel 1**

Herstellung eines hochgereinigten Protein C-Aktivatorpräparates aus A. contortrix-Gift.

1 g A. contortrix-Gift wird in 100 ml Wasser gelöst, der pH-Wert dieser Lösung mit 0-Phosphorsäure 0,3 mol/l auf 3,0 gestellt und die saure Giftlösung während 10 Minuten in einem Wasserbad bei 70 ± 2°C gehalten, anschließend auf 20°C gekühlt, der pH-Wert mit Natronlauge (1 mol/l) auf 7,2 gestellt, die trübe Lösung zentrifugiert und der Überstand mit destilliertem Wasser auf ein Volumen von 100 ml verdünnt, um so eine vorgereinigte Giftfraktion zu erhalten.

Die vorgereinigte Giftfraktion wird auf eine mit 0,015 mol/l Natriumphosphatpuffer, pH 6,8, äquilibrierte Säule mit DEAE-Sephadex® A-50 vom Format 2,6 x 90 cm aufgetragen und mit einem linearen Gradienten, gemischt aus 0,015 mol/l Natriumphosphatpuffer, pH 6,8, und 0,4 mol/l Natriumchlorid in 0,015 mol/l Natriumphosphatpuffer, pH 6,8, eluiert und Fraktionen von je 20 ml gesammelt. Die Protein C-aktivierende Wirkung der einzelnen Fraktionen wird dadurch ermittelt, daß man 0,1 ml menschliches Citratplasma mit 0,1 ml Probe (1:350 in Wasser verdünnte Fraktion) und 0,1 ml Probe

Ellagsäurereagenz (Actin®) und 0,1 ml 0,025 mol/l Calciumchloridlösung versetzt, sofort eine Stopuhr in Gang bringt und die Zeit bis zur Gerinnung bestimmt. Die Protein C-Aktivator enthaltenden Proben bewirkten eine Verlängerung der Gerinnungszeit von 34 Sekunden auf bis zu 200 Sekunden, je nach Aktivatorgehalt.

Die Protein C aktivierenden Fraktionen werden vereinigt, durch Ultrafiltration auf 1/10 vom Eluatvolumen eingeengt, in 0,05 mol/l Natriumacetatpuffer, pH 5,0, aufgenommen und auf eine mit 0,01 mol/l Natriumacetatpuffer, pH 5,0, äquilibrierte Säule von CM-Sephadex® C-50 aufgetragen, und mit einem linearen Gradienten, gemischt aus 0,05 mol/l Natriumacetatpuffer, pH 5,0 und 0,4 mol/l Natriumchlorid in 0,05 mol/l Natriumacetatpuffer, pH 5,0, eluiert und Fraktionen von je 20 ml gesammelt, welche nach der oben beschriebenen Methode auf Protein C aktivierende Wirkung geprüft werden.

Die Protein C aktivierenden Fraktionen werden zusammen gemischt, durch Ultrafiltration auf 1/25 ihres Volumens eingeengt, mit 1 % Essigsäure in dest. Wasser auf 25 ml gestellt und auf eine mit 1 % Essigsäure in Wasser äquilibrierte Säule von Sephadex® G-100 aufgetragen, mit 1 % Essigsäure eluiert und Fraktionen von je 20 ml gesammelt, die wiederum nach der eingangs beschriebenen Methode auf Protein C-aktivierende Wirkung geprüft werden.

Die Protein C-aktivierenden Fraktionen werden vereinigt und lyophilisiert. Es wird ein salzfreies Aktivatorpräparat erhalten, welches in der Polyacrylamidgel-Elektrophorese eine einzige Zone und eine Protein C-aktivierende Aktivität von 35 U per mg aufweist.

Eine Einheit (U) Protein C-Aktivator ist die Menge, welche die in 1 ml normalem, menschlichem Citratplasma enthaltene Menge Protein C vollständig aktiviert.

## Beispiel 2

Messung von aktiviertem Protein C mittels Verlängerung der partiellen Thromboplastinzeit (in Anwesenheit von Protein C-Aktivator nach Beispiel 1).

25 $\mu$l verdünnte Plasmaprobe (1+4 mit 0,9%iger Natriumchloridlösung) + 25 $\mu$l Protein C-defektes Normalplasma und 500 $\mu$l Reagenz, bestehend aus Kephalin und Ellagsäure in 10 mmol/l Tris/HCl, pH 7,6, werden in einer Plastikküvette bei 37°C inkubiert. Nach genau 1 Minute werden entweder

1. 20 $\mu$l Protein C-Aktivator nach Beispiel 1 (Konzentration: Flascheninhalt = 3 U in 3 ml 0,9%iger NaCl-Lösung) oder

2. 20 $\mu$l 0,9%ige NaCl-Lösung zugegeben, gemischt und weiter inkubiert.

Nach genau 4 Minuten werden 150 $\mu$l Startreagenz, bestehend aus 1,1 mmol/l Chromozym® TH • (Tos-Gly-Pro-Arg-pNA) und 100 mmol/l Calciumacetat, zugegeben und bei 405 nm in einem Photometer die Zeit bestimmt, bis eine definierte Menge Substrat von neu entstandenem Thrombin zu Tos-Gly-Pro-Arg und para-Nitroanilin (pNA) umgesetzt ist. Die Menge umgesetzten Substrats, die gemessen wird, wird durch einen Schwellenextinktionswert (z. B. $\Delta E$ = 0,2) definiert.

Unter den angegebenen Bedingungen nimmt die partielle Thromboplastinzeit proportional mit der Konzentration an Protein C in der Plasmaprobe zu, wenn dem Gemisch der Aktivator Protac zugegeben wurde. Da die partielle Thromboplastinzeit unter anderem vom Faktorengehalt des Plasmas abhängt, ist es zweckmäßig, einen Leerwert mit 0,9%iger NaCl-Lösung mitzuvermessen. Das Verhältnis aus PTT-Zeit mit Aktivator zu PTT-Zeit ohne Aktivator ist ein Maß für den Protein C-Gehalt. Über eine Eichkurve, die mit Plasmaproben unterschiedlicher, bekannter Protein C-Konzentration erhalten wird, kann die Protein C-Konzentration der unbekannten Plasmaprobe bestimmt werden. In der Tabelle sind drei Beispiele, Plasmaproben unbekannter Protein C-Konzentrationen, enthalten.

Tabelle I

| Verlängerung der PTT-Zeit durch aktiviertes Protein C in Plasmaverdünnungen und Plasmaproben | | | |
|---|---|---|---|
| Probe | Antigen* | Verhältnis mit/ohne Protein C-Aktivator nach Beispiel 1 | Aktivität |
| Standard 1 | 125 % | 2,01 | 125 % |
| Standard 2 | 100 % | 1,86 | 100 % |
| Standard 3 | 75 % | 1,68 | 75 % |
| Standard 4 | 50 % | 1,56 | 50 % |
| Standard 5 | 25 % | 1,32 | 25 % |
| Standard 6 | 10 % | 1,17 | 10 % |
| PC defektes Plasma | 0 % | 1,04 | 0 % |
| Kontrollplasmen | 114 % | 1,94 | 114 % |
|  | 81 % | 1,70 | 75 % |
|  | 23,5 % | 1,27 | 22 % |

\* mit ELISA-Test von BM

**Patentansprüche**

1. Verfahren zur photometrischen Bestimmung von Protein C- oder Protein S-Aktivität, insbesondere im Plasma,
   **dadurch gekennzeichnet,**
   daß die das zu bestimmende Protein C oder Protein S enthaltende Probe mit einem Protein C-Aktivator aus Schlangengift unter Bildung von aktiviertem Protein C oder Protein S inkubiert wird, ein Aktivator für Faktor XII, für Faktor VII oder für Faktor II zugesetzt wird und die Abnahme der durch Gerinnungsfaktoren und deren Aktivatoren vermittelten Bildung von Thrombin aus Prothrombin mittels eines chromogenen Thrombinsubstrates bestimmt wird.

2. Verfahren nach Anspruch 1, **dadurch gekennzeichnet,** daß man als Protein C-Aktivator eine Lösung des Gifts der Schlange Agkistrodon contortrix contortrix oder von Komponenten desselben verwendet.

3. Verfahren nach Anspruch 1, **dadurch gekennzeichnet,** daß man als Protein-C-Aktivator Gift der Schlangen A.C. mokasen, der A.C. pictigaster,der A. piscivours, der A.p. leucostoma, der A. bilineatus, der Bothrops moojeni, der B. pradoi, der Cerastes cerastes, der Vipera lebetrina oder der V. russellii verwendet.

4. Verfahren nach Anspruch 1, 2 oder 3, **dadurch gekennzeichnet,** daß man als chromogenes Thrombinsubstrat H-D-Phe-Pip-Arg-pNA oder Tos-Gly-Pro-Arg-pNA verwendet.

5. Verfahren nach einem der Ansprüche 1 bis 4, **dadurch gekennzeichnet,** daß man einen Aktivator für Faktor XII zusetzt.

6. Verfahren nach Anspruch 5, **dadurch gekennzeichnet,** daß man Kephalin und Ellagsäure zusetzt.

7. Verfahren nach einem der Ansprüche 1 bis 4, **dadurch gekennzeichnet,** daß man einen Aktivator für Faktor VII zusetzt.

8. Verfahren nach Anspruch 7, **dadurch gekennzeichnet,** daß man Faktor V sowie als Faktor VII-Aktivator Thromboplastin zusetzt.

9. Verfahren nach einem der Ansprüche 1 bis 4, **dadurch gekennzeichnet,** daß man einen Aktivator für Faktor II zusetzt.

10. Verfahren nach Anspruch 9, **dadurch gekennzeichnet,** daß man Kephalin und Faktor Xa zusetzt.

**11.** Verfahren nach einem der Ansprüche 1 bis 10, **dadurch gekennzeichnet,** daß man Protein C-Mangelplasma zusetzt.

**12.** Verfahren nach einem der Ansprüche 1 bis 10, **dadurch gekennzeichnet,** daß man Protein S-Mangelplasma zusetzt.

**Claims**

**1.** Process for the photometric determination of protein C and/or protein S activity, especially in plasma, characterised in that the sample containing the protein C or protein S to be determined is incubated with a protein C activator from snake venom with the formation of activated protein C or protein S, an activator for Factor XII, for Factor VII or for Factor II is added thereto and the decrease of the formation of thrombin from prothrombin brought about by coagulation factors and their activators is determined by means of a chromogenic thrombin substrate.

**2.** Process according to claim 1, characterised in that, as protein C activator, one uses a solution of the venom of the snake Agkistrodon contortrix contortrix or of components thereof.

**3.** Process according to claim 1, characterised in that, as protein C activator one uses venom of the snakes A. C. mokasen, of the A. C. pictigaster, of the A. piscivours, of the A. p. leucostoma, of the A. bilineatus, of the Bothrops moojeni, of the B. pradoi, of the Cerastes cerastes, of the Vipera lebetrina or of the V. russellii.

**4.** Process according to claim 1, 2 or 3, characterised in that, as chromogenic thrombin substrate, one uses H-D-Phe-Pip-Arg-pNA or Tos-Gly-Pro-Arg-pNA.

**5.** Process according to one of claims 1 to 4, characterised in that one adds an activator for Factor XII.

**6.** Process according to claim 5, characterised in that one adds cephalin and ellagic acid.

**7.** Process according to one of claims 1 to 4, characterised in that one adds an activator for Factor VII.

**8.** Process according to claim 7, characterised in that one adds Factor V, as well as Factor VII activator thromboplastin.

**9.** Process according to one of claims 1 to 4, characterised in that one adds an activator for Factor II.

**10.** Process according to claim 9, characterised in that one adds cephalin and Factor Xa.

**11.** Process according to one of claims 1 to 10, characterised in that one adds protein C-deficient plasma.

**12.** Process according to one of claims 1 to 10, characterised in that one adds protein S-deficient plasma.

**Revendications**

**1.** Procédé de détermination photométrique de l'activité de la protéine C ou de la protéine S, en particulier dans le plasma, caractérisé en ce que l'échantillon contenant la protéine C ou la protéine S à déterminer est incubé avec un activateur de la protéine C provenant de venin de serpent avec formation de protéine C ou de protéine S activée, un activateur du facteur XII, du facteur VII ou du facteur II est ajouté et la diminution de la formation de thrombine à partir de prothrombine provoquée par des facteurs de coagulation et leurs activateurs est déterminée à l'aide d'un substrat chromogène de la thrombine.

**2.** Procédé selon la revendication 1, caractérisé en ce que l'on utilise comme activateur de la protéine C une solution de venin du serpent Agkistrodon contortrix contortrix ou de composants de celui-ci.

**3.** Procédé selon la revendication 1, caractérisé en ce que l'on utilise comme activateur de la protéine C le venin des serpents A.C. mokasen, A.C. pictigaster, A. piscivorus, A.p. leucostoma, A. bilineatus, Bothrops moojeni, B. pradoi, Cerastes cerastes, Vipera lebetrina ou V. russellii.

**4.** Procédé selon la revendication 1, 2 ou 3, caractérisé en ce que l'on utilise comme substrat chromogène de la thrombine H-D-Phe-Pip-Arg-pNA ou Tos-Gly-Pro-Arg-pNA.

**5.** Procédé selon l'une des revendications 1 à 4, caractérisé en ce que l'on ajoute un activateur du facteur XII.

**6.** Procédé selon la revendication 5, caractérisé en ce que l'on ajoute de la céphaline et de l'acide ellagique.

**7.** Procédé selon l'une des revendications 1 à 4, caractérisé en ce que l'on ajoute un activateur du facteur VII.

**8.** Procédé selon la revendication 7, caractérisé en ce que l'on ajoute du facteur V ainsi que de la thromboplastine comme activateur du facteur VII.

**9.** Procédé selon l'une des revendications 1 à 4, caractérisé en ce que l'on ajoute un activateur du facteur II.

**10.** Procédé selon la revendication 9, caractérisé en ce que l'on ajoute de la céphaline et du facteur Xa.

**11.** Procédé selon l'une des revendications 1 à 10, caractérisé en ce que l'on ajoute un plasma exempt de protéine C.

**12.** Procédé selon l'une des revendications 1 à 10, caractérisé en ce que l'on ajoute un plasma exempt de protéine S.

**Fig.1** Eichkurve für Protein C –Aktivitätsbestimmung.
Das Verhältnis der partiellen Thromboplastinzeit mit
bzw. ohne Aktivator(=ratio) ist gegen die Prozent –
konzentration von Protein C in Plasma aufgetragen.

$$ratio = \frac{PTT + Aktivator}{PTT\emptyset\, Aktivator}$$